# FASCICULE DE BREVET EUROPEEN

(11) **EP 3 204 483 B1**
(45) Date de publication et mention de la délivrance du brevet: **24.04.2019**
(21) Numéro de dépôt: 15778302.8
(22) Date de dépôt: 09.10.2015
(51) Int. Cl.: C12J 1/00, C12G 1/022, C12G 3/02, C12H 1/00, C12G 3/07, C12J 1/02, C12J 1/08, C12N 1/00, C12N 1/16, C12N 1/22, C12P 7/00, C12P 7/12, C12P 7/14, C12C 11/09, C12G 1/02

(54) **PROCÉDÉ DE PRÉPARATION DE VIN UTILISANT DES BIOFILMS BACTÉRIENS**
VERFAHREN ZUR HERSTELLUNG VON WEIN MITHILFE VON BAKTERIELLEN BIOFILMEN
PROCESS FOR THE PREPARATION OF WINE USING BACTERIAL BIOFILMS

(30) Priorité: 10.10.2014 FR 1459736
(43) Date de publication de la demande: 16.08.2017
(73) Titulaire: Université de Bourgogne, 21000 Dijon (FR); Institut National Supérieur des Sciences Agronomiques, de l'Alimentation et de l'Environnement, 21000 Dijon (FR)
(72) Inventeur: GUZZO, Jean, F-21121 Fontaine-lès-Dijon (FR); WEIDMANN-DESROCHE, Stéphanie, F-21800 Quétigny (FR); BASTARD, Alexandre, F-21000 Dijon (FR); COELHO, Christian, F-63720 Entraigues (FR); GOUGEON, Régis, F-21800 Quétigny (FR)
(74) Mandataire: Regimbeau
(86) Numéro de dépôt international: PCT/EP2015/073343
(87) Numéro de publication internationale: WO 2016/055603

(56) Documents cités:
- WO-A1-2007/063228
- FR-A1- 2 856 074
- NEL ET AL: "Effect of Bacteriocins Pediocin PD-1, Plantaricin 423, and Nisin on Biofilms of Oenococcus oeni on a Stainless Steel Surface", AM J ENOL VITIC, vol. 53, no. 3, 1 janvier 2002 (2002-01-01), pages 191-196, XP055036386,
- LENGELER J W: 1 janvier 1999 (1999-01-01), BIOLOGY OF THE PROKARYOTES, XP008176780, ISBN: 0-632-05357-7 pages 723-762, le document en entier

## Description

### DOMAINE DE L'INVENTION

La présente invention concerne un procédé de préparation des vins comprenant le déclenchement de la fermentation malolactique par inoculation avec des bactéries lactiques du genre *Oenococcus,* sous forme de biofilm.

### ETAT DE LA TECHNIQUE

De nos jours, dans le domaine de l'oenologie moderne, la maîtrise des différentes étapes de l'élaboration du vin nécessite l'utilisation de ferments levuriens pour la fermentation alcoolique et bactériens pour la fermentation malolactique. La fermentation malolactique intervient en général après la fermentation alcoolique. Elle consiste en une transformation de l'acide malique en acide lactique par l'intermédiaire de bactéries appelées bactéries lactiques. La fermentation malolactique se traduit par une diminution de l'acidité permettant une stabilisation et un assouplissement du vin ainsi que le maintien d'une qualité constante d'une année à l'autre.

La fermentation malolactique influençant les qualités organoleptiques des vins, elle doit être rigoureusement contrôlée. La fermentation spontanée pouvant se déclencher à tout moment du processus il a été développé des moyens pour déclencher cette fermentation au moment opportun. Ainsi, des bactéries lactiques dites « prêtes à l'emploi » ont été développées. Ces bactéries permettent d'améliorer la maitrise de la conduite de la fermentation malolactique et de produire des vins de qualité constante avec une certaine typicité répondant à la demande des consommateurs. *Oenococcus oeni* est l'espèce bactérienne la plus fréquemment utilisée et la mieux adaptée pour réaliser la fermentation malolactique. Ainsi, par rapport à une fermentation malolactique aléatoire réalisée avec des bactéries lactiques indigènes, l'ensemencement bactérien présente de nombreux avantages.

Cependant, les conditions physico-chimiques du vin (pH acide, forte teneur en éthanol, présence de sulfite, basse température, carence nutritionnelle) étant défavorables à l'implantation de ces ferments lactiques lors de l'inoculation, il a été nécessaire de mettre au point des protocoles industriels de pré-acclimatation aux conditions du vin pour pallier ces problèmes de manière à optimiser la survie et la reprise de croissance des bactéries dans le vin après inoculation directe.

Par exemple, le brevet WO 2007/063228 décrit un procédé de prédiction de la viabilité, de la vitalité et de la stabiilté de bactéries destinées à être utilisées dans des environnements stressants.

Le brevet FR 2 858 074 décrit également un procédé de contrôle de la fermentation malolactique dans les vins par inoculation directe de souches de bactéries lactiques capables de se développer dans un milieu contenant de l'alcool.

En particulier, la société Lallemand S.A.S. utilise des protocoles de pré-acclimatation nommés MBR® ou **1-STEP**®. La fermentation malolactique se réalise souvent en fût. Les ferments malolactiques y sont introduits sous forme congelés ou lyophilisés et se développent sous forme planctonique. Les souches bactériennes ainsi acclimatées restent onéreuses pour le vinificateur.

Un besoin demeure donc pour la mise au point d'un procédé simple à mettre en oeuvre permettant de déclencher la fermentation, en particulier la fermentation malolactique, de manière reproductible et à moindre coût. De manière avantageuse, le procédé permettra de moduler les propriétés organoleptiques des boissons fermentées ainsi préparées.

### BREVE DESCRIPTION DE L'INVENTION

La présente invention porte sur un procédé de préparation d'un vin qui comprend le déclenchement de la fermentation par inoculation de la boisson fermentescible avec des bactéries fermentaires du genre *Oenococcus* sous forme de biofilm.

### BREVE DESCRIPTION DES FIGURES

La figure 1 représente la survie des cellules de *O. oeni* en fonction du temps dans un milieu de croissance (MRSm) modifié par ajout d'éthanol et abaissement du pH à 3,0 afin de générer des conditions stressantes.
La figure 2A représente l'activité malolactique des cellules de *O. oeni* cultivées auparavant soit en biofilm (courbe avec carré) soit en planctonique (courbe avec cercle) et incubées dans un jus de raisin fermenté standardisé à 12% d'éthanol, 4 g/l d'acide L-malique, pH3,5.
La figure 2B représente l'activité malolactique des cellules de O. *oeni* cultivées auparavant soit en biofilm (courbe avec carré) soit en planctonique (courbe avec cercle) et incubées dans un jus de raisin fermenté standardisé à 12% d'éthanol, 4 g/l d'acide L-malique, pH3,2.
La figure 3 représente les dosages (concentration en µg/l) des 6 arômes marqueurs du bois (du haut à gauche au bas à droite : Furfural, Vanilline, Guaiacol, Eugénol, cis-WhiskyLactone, trans-WhiskyLactone) dans des vins de différentes conditions : Barre de gauche : « Chêne » (contact bois-vin, sans activité microbiologique),
   Barre centrale : « FML + chêne » (fermentation malolactique par des bactéries planctoniques pendant le temps de contact bois-vin),
   Barre de droite : « Biofilm » (biofilm formé sur bois de chêne, effectuant la fermentation malolactique, libérant des arômes du bois dans le vin).
   Les différences significatives (P=0,05) entre les conditions sont mentionnées par des lettres différentes.

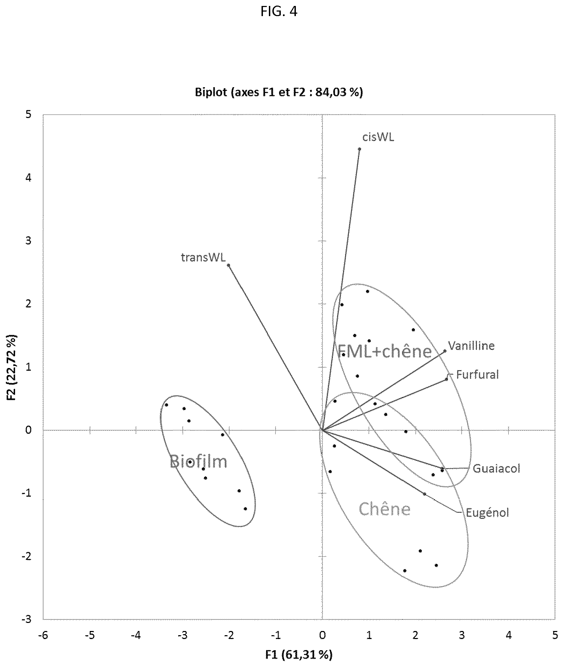

La figure 4 représente l'analyse en composantes principales (84%) de la projection du jeu de données des dosages des 6 arômes marqueurs du bois dans des vins de différentes conditions : « Chêne » (contact bois-vin, sans activité microbiologique), « FML + chêne » (fermentation malolactique réalisée par des bactéries planctoniques pendant le temps de contact bois-vin), « Biofilm » (biofilm formé sur bois de chêne, effectuant la fermentation malolactique, libérant des arômes du bois dans le vin).

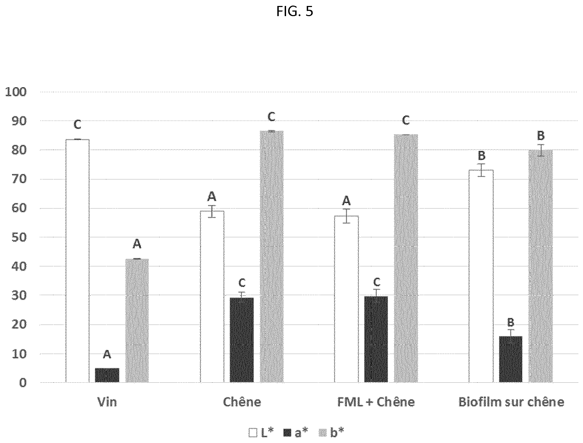

La figure 5 représente la mesure de la couleur par les paramètres L* a* b* (respectivement histogrammes blanc, noir et gris) des quatre vins de différentes conditions (de gauche à droite):
   « Vin » (vin initial avant expérimentation),
   « Chêne » (contact bois-vin, sans activité microbiologique),
   « FML + chêne » (fermentation malolactique réalisée par des bactéries planctoniques pendant le temps de contact bois-vin),
   « Biofilm » (biofilm formé sur bois de chêne, effectuant la fermentation malolactique, libérant des arômes du bois dans le vin).
   Les différences significatives (P=0,05) entre les conditions sont mentionnées par des lettres différentes.

### DESCRIPTION DETAILLEE DE L'INVENTION

Les inventeurs ont mis en évidence que l'inoculation de boissons fermentescibles avec des bactéries fermentaires sous forme de biofilm permet de déclencher la fermentation de manière reproductible, à moindre coût et sans difficulté de mise en oeuvre. En particulier, les inventeurs ont mis en évidence que l'inoculation de boissons fermentescibles avec des bactéries lactiques sous forme de biofilm permet de déclencher la fermentation malolactique de manière reproductible, à moindre coût et sans difficulté de mise en oeuvre. De manière avantageuse, dans certains modes de réalisation, l'inoculation de boissons fermentescibles avec des bactéries lactiques sous forme de biofilm permet de moduler les propriétés organoleptiques des boissons fermentées ainsi préparées.

La présente description porte ainsi sur un procédé de préparation d'une boisson fermentée comprenant le déclenchement de la fermentation par inoculation de la boisson fermentescible avec des bactéries fermentaires sous forme de biofilm. En particulier, la présente description porte sur un procédé de préparation d'une boisson fermentée comprenant le déclenchement de la fermentation malolactique par inoculation de la boisson fermentescible avec des bactéries lactiques sous forme de biofilm.

La présente invention porte sur un procédé de préparation d'un vin comprenant le déclenchement de la fermentation par inoculation de la boisson fermentescible avec des bactéries fermentaires du genre *Oenococcus* sous forme de biofilm.

Le vin peut être choisie parmi les vins rouges, les vins blancs, les vins rosés et les champagnes.

Dans certains modes de réalisation, le procédé de la présente invention comprend l'inoculation de la boisson fermentescible avec une combinaison de bactéries fermentaires et de levures fermentaires sous forme de biofilm. Il doit être compris que le biofilm comprend les bactéries et les levures fermentaires en combinaison. Un tel biofilm est dit « mixte ». Les bactéries fermentaires appartiennent au genre *Oenococcus.* Les levures peuvent être choisies parmi les souches appartenant au genre *Sacharomyces, Schizosaccharomyces Brettanomyces, Torulaspora, Candida, Metschnikowia, Kluyveromyces* ou leurs combinaisons. La co-inoculation permet avantageusement de déclencher l'enchainement de la fermentation alcoolique et de la fermentation malolactique. Elle peut permettre également de développer les flaveurs du vin produite.

Ainsi, dans le procédé de la présente invention, la fermentation peut être déclenchée par inoculation du vin avec des bactéries fermentaires, qui peuvent être de même souche ou être une combinaison de deux ou plusieurs souches de la même espèces, sous forme de biofilm ou être déclenchée par inoculation avec des bactéries et des levures fermentaires, les bactéries et les levures étant en combinaison sous la forme d'un biofilm. De tels biofilms sont dits « biofilms mixtes » (combinaisons de bactéries et de levure).

Les bactéries fermentaires sous forme de biofilm, ainsi que les levures quand présentes, ont l'avantage d'être plus résistantes aux stress (faible pH, fort taux d'éthanol ; par exemple pour le vin, pH inférieur à 3,5, concentration en éthanol supérieure ou égale à 12% volumique, ...) par rapport aux bactéries, et le cas échéant levures, ne se présentant pas sous une telle organisation. Il n'apparait donc plus nécessaire de pré-acclimater les bactéries aux conditions physicochimiques des boissons à fermenter, ce qui permet de réduire considérablement les coûts liés à la préparation de bactéries, optionnellement en combinaison avec des levures, « prêtes à l'emploi ».

Les biofilms sont des structures tridimensionnelles constituées par des populations bactériennes englobées dans une matrice exopolymérique (polysaccharides, polypeptides, acides nucléiques). Ils se développent sur différents types de support. Souvent considérés comme néfastes du fait qu'ils peuvent constituer une réserve de micro-organismes et être la source de contaminations récurrentes du milieu environnant, les biofilms sont jusqu'à présent encore faiblement valorisés. L'attachement des bactéries sous forme de biofilms est décrite dans un cadre très général dans l'extrait de Lengeler, J.W., « Biology of the prokaryotes », pages 723-762, 1999.

De manière générale, la formation des biofilms résulte d'un processus dynamique et complexe. Le biofilm se constitue en plusieurs étapes depuis l'adhésion initiale à un support à la formation d'une structure tridimensionnelle qui peut constituer un véritable écosystème assurant des fonctions diverses. La matrice exopolymérique est un élément clé du biofilm et constitue une véritable barrière physique et biologique qui protège les bactéries des stress environnementaux (agents antimicrobiens, stress acides, thermiques, ...) mais aussi joue le rôle de filtre en alimentant en nutriments le biofilm. Les bactéries présentes dans le biofilm bénéficient de cet environnement propice pour proliférer et développent un phénotype « Biofilm » qui leur confère une très forte résistance au stress et des fonctionnalités spécifiques (Rieu *et al.,* 2014).

Le biofilm peut être préparé par incubation des micro-organismes dans un milieu favorable à leur croissance en présence du support solide. Le temps de croissance peut dépendre de l'espèce et au sein d'une espèce de la souche utilisée. Par exemple, dans le cas de la souche *O. oeni* ATTCC BBA-1163, l'incubation dure au maximum 7 jours en milieu MRS modifié.

Les inventeurs ont montré que les bactéries fermentaires sous forme de biofilm, en particulier les cellules de *O. oeni* cultivées en biofilm, présentent une résistance accrue au stress (faible pH, fort taux d'éthanol ; par exemple pour le vin, pH inférieur à 3,5, concentration en éthanol supérieure ou égale à 12% volumique, ...) par rapport aux cellules cultivées en milieu liquide. Cette résistance au stress accrue leur permet de résister aux conditions physico-chimiques défavorables pouvant être rencontrées lors de la préparation des boissons fermentées, en particulier du vin.

Par ailleurs, les performances malolactiques des bactéries sous forme de biofilm sont supérieures à celles de bactéries communément cultivées en planctonique.
Le phénotype acquis par les bactéries au cours de la croissance en biofilm leur confère cette résistance qui aboutit à de meilleures performances pour utiliser le malate et donc réaliser la fermentation malolactique du vin dans un temps raccourci.

Ainsi, les bactéries et levures sous forme de biofilm présentent une résistance accrue au stress. Par ailleurs, les constituants de la matrice exopolymérique du biofilm peuvent avantageusement permettre de moduler les qualités organoleptiques et flaveurs de la boisson fermentée préparée. Ainsi, le procédé de la présente invention peut permettre la préparation et la modulation des qualités organoleptiques d'une boisson fermentée par inoculation de la boisson fermentescible avec des bactéries fermentaires, et éventuellement levures, sous forme de biofilm. En particulier, le procédé de la présente invention peut permettre de moduler le transfert des molécules aromatiques du bois vers la boisson fermentée et/ou de moduler le transfert des pigments du bois vers la boisson fermentée.

Les constituants de la matrice exopolymérique du biofilm pouvant, en tant que tels, moduler les qualités organoleptiques des boissons (fermentées ou non), ceux-ci peuvent être utilisés, après extraction du biofilm, dans des procédés pour moduler les qualités organoleptiques des boissons. Ainsi, la présente invention porte également sur l'utilisation des constituants, en particulier les exopolysaccharides, de la matrice exopolymérique d'un biofilm de bactéries, et éventuellement de levures, pour moduler les qualités organoleptiques d'une boisson.

Les bactéries fermentaires sous forme de biofilm ou les combinaisons de bactéries et levures fermentaires sous forme de biofilm peuvent être inoculées sous une forme décrochée du support, c'est-à-dire après décrochage du support ayant permis le développement du biofilm, ou sous une forme adhérente au support, c'est-à-dire encore accrochées au support ayant permis le développement du biofilm. Le support peut être tout support abiotique adapté. Par exemple, Nel et al. (Am. J. Enol. Vitic. 2002, 53, 191-196) décrivent des biofilms de Oenococcus oeni sur une surface d'acier inoxydable utilisé, par inoculation par exemple d'un moût de Chardonnay. Dans le cadre de la présente invention le support peut en particulier être choisi parmi le bois, tout particulièrement choisi parmi les essences de bois utilisées comme contenant de produit liquide fermenté, le liège, l'acier inoxydable, le polystyrène, le silicone ou les composites de polyéthylène. Le support bois pourra se présenter sous différentes formes, par exemple des copeaux de bois. Lorsque le support est du bois, celui-ci peut être chauffé ou non chauffé. Le support se présente typiquement sous forme de plaquettes (parallélépipèdes) ou de granulats.

Les bactéries fermentaires sous forme de biofilm ou les combinaisons de bactéries et levures fermentaires sous forme de biofilm, décrochées du support ou adhérentes, peuvent se présenter sous différentes formes qui préservent leur viabilité et vitalité, telles que sous vide, congelée ou lyophilisée.

Les bactéries fermentaires, ou les combinaisons de bactéries et levures fermentaires détachées du biofilm peuvent, en particulier, se présenter sous forme congelée ou sous forme lyophilisée.

Les bactéries fermentaires, ou les combinaisons de bactéries et levures fermentaires en biofilm (adhérentes) peuvent se présenter sous forme congelée, sous forme lyophilisée, sous vide, sous atmosphère contrôlée ou encore sous forme native.

La matrice exopolymérique purifiée du biofilm peut se présenter sous forme congelée, lyophilisée, sous vide, sous atmosphère inerte, ou sous forme native et peut être ajoutée pour développer les qualités organoleptiques des boissons fermentées ou non fermentées. L'homme du métier saura déterminer la quantité de bactéries fermentaires devant être inoculées afin de déclencher la fermentation, cette quantité variant en fonction de la nature de la boisson à fermenter et des bactéries fermentaires choisies.

Typiquement, un minimum de 10⁶ à 10⁷ UFC/mL. est employé pour déclencher la fermentation malolactique lors de la préparation des vins. De manière avantageuse, lorsque le support est du bois et que les bactéries, éventuellement en combinaison avec des levures, sont inoculées sous forme adhérente au support, l'inoculation peut permettre de moduler les propriétés organoleptiques des boissons fermentées préparées.

Il est bien connu qu'au cours du vieillissement du vin en fût, des transferts ont lieu du bois vers le vin mais aussi du vin vers le bois. Les quantités et les composés qui migrent, dépendent des caractéristiques du bois (espèce, conditions de séchage, traitement thermique, nombre d'utilisation...) et du vin (pH, cépages utilisés, levures, bactéries, composition chimique...). L'extraction de composés du bois par le vin est le sujet de nombreuses recherches. La meilleure compréhension de ce phénomène permet au maitre de chai de déterminer la durée de l'élevage en fût. La simple extraction des composés aromatiques (volatils et polyphénols) et des tanins du bois ajoute de la richesse et de la complexité aux arômes des vins (Díaz-Plaza *et al.,* 2002, Perez-Prieto *et al.,* 2002 ; Fernandez de Simón *et al.,* 2003). Une grande variété de composés aromatiques appartenant à différentes familles chimiques a été identifiée dans le bois de chêne (Cadahía *et al.,* 2003 ; Fernandez de Simón *et al.,* 2006). Les ellagitanins (tanins du bois) ont un rôle important d'antioxydants, et grâce à leur capacité à consommer de grandes quantités d'oxygène, ils vont réguler le procédé d'oxydation du vin. L'extraction des principaux composés volatils provenant de la dégradation des composants du bois pendant la maturation et la chauffe peut augmenter la complexité aromatique des vins vieillis en fût (Cadahía et al., 2003 ; Fernandez de Simón et al., 2006). Après leur extraction, ces composés subissent une série de modifications biochimiques qui ont un impact sur les propriétés sensorielles du vin.

**Tableau 1 : Descripteurs d'odeur de différents extraits de bois**

| **Composés du bois de chêne** | **Descripteurs olfactifs** |
|---|---|
| **Whisky lactone** | Vanilline, boisé, clou de girofle, noix de coco |
| **Furfural** | Légèrement grillé, caramel |
| **5-methylfurfural** | Epicé, grillé, sucré |
| **Gaïacol** | Epicé, grillé, fumé/brûlé |
| **Eugénol** | Epicé, clou de girofle, cannelle |
| **Isoeugénol** | Epicé, clou de girofle, boisé |
| **Vanilline** | Sucré, vanille |

Les copeaux de bois et les « staves » (des morceaux de douelles), sont des alternatives à l'utilisation des fûts mais elles ne sont pas autorisées par toutes les législations. Les copeaux de bois sont principalement utilisés pour accélérer l'élaboration du vin par l'acquisition de notes boisées car ce format augmente les surfaces d'échange, permettant des transferts plus rapides et plus importants pour la même masse de bois (Fernández de Simón *et al.,* 2010).

L'inoculation de bactéries sous forme adhérente à un support de bois permet ainsi de développer les propriétés organoleptiques des boissons préparées, en particulier des vins, à la manière des copeaux de bois utilisés en alternative à l'utilisation des fûts.

La présente description porte également sur une boisson fermentée susceptible d'être obtenues par le procédé de la présente invention tel que décrit ci-dessus. De telles boissons peuvent présenter des propriétés organoleptiques distinctives.

### EXEMPLES

### Souche bactérienne

Pour toutes les expérimentations la souche utilisée est *Oenococcus oeni* ATTCC BBA-1163.

### Les milieux de culture des bactéries utilisés dans cette étude sont les suivants :

**MRSm** : 52 g/l MRS (Conda) auquel est ajouté : 10 g/l Fructose ; 8 g/l Acide (D-L) Malique. Le pH est ajusté à 4,8.

**MRSstress** : MRSm auquel est ajouté 13% d'éthanol, pH ajusté à 3,0.

**Vin** : un jus de raisin blanc commercial est fermenté grâce à une LSA (levure sèche active) puis ses paramètres sont standardisés : pH à 3,2 ou 3,5 ; acide L-malique à 4 g/l ; éthanol à 12% volumique. Le vin est ensuite filtré à 0,22µm.

### 1. Survie des cellules en condition de stress acide et en présence d'éthanol en fonction des conditions de culture biofilm ou planctonique.

Les cellules planctoniques sont issues d'une culture de 24h en MRSm à 28°C. Le biofilm est obtenu par une culture de 1 semaine en MRSm sur coupon d'acier (2,5*2,5 cm) à 28°C. Les cellules sessiles (biofilm) sont décrochées mécaniquement de l'acier et incubées en conditions de stress (Milieu MRSstress). Les cellules vivantes et cultivables sont dénombrées sur gélose MRSm après 0, 1, 4 et 24 h d'incubation en MRSstress.

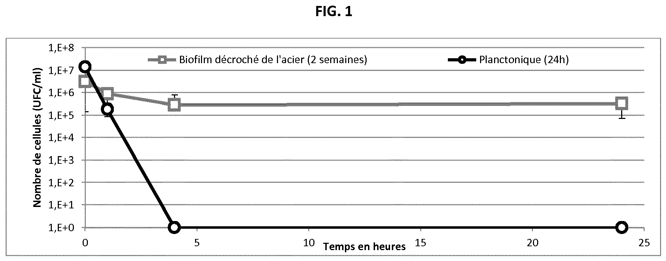

Les résultats sont présentés à la figure 1. Carré: cinétique de survie des cellules planctoniques ; cercle : cinétique de survie des cellules décrochées d'un biofilm sur acier (2 semaines). L'incubation a lieu en MRSstress (13% d'éthanol, pH3).

Les conditions de stress utilisées dans cette expérience correspondent à un environnement extrêmement stressant pour la bactérie qui peut correspondre aux conditions présentes dans certains vins. Les cellules planctoniques ensemencées à 10⁷UFC/ml dans ce milieu ne survivent pas avec une mortalité totale au bout de 4 heures. Les cellules cultivées auparavant en biofilm ont une perte d'un log au bout de 4 heures d'incubation. De plus la viabilité se maintient constante sur 24h.

Cette première expérience montre que les cellules de *O. oeni* cultivées en biofilm acquièrent une résistance au stress qui leur confère la capacité de survivre à des stress rencontrés dans le vin, ces conditions de stress étant normalement létales pour des cellules cultivées en milieu liquide (planctonique).

### 2. Suivi de la fermentation malolactique réalisée avec des cellules O. oeni cultivées en biofilm ou en planctonique.

Les cellules planctoniques sont cultivées en MRSm à 28°C. Le biofilm est obtenu par une culture de 7 jours en MRSm sur coupon de bois (2,5*2,5 cm). Dans le cas des biofilms, le milieu est renouvelé une fois à la moitié du temps (3,5 j). La fermentation malolactique est mise en oeuvre par l'ensemencement direct de la culture planctonique (8.10⁷ UFC/ml) dans 20 ml de vin ou immersion du biofilm sur coupon de bois (5.10⁷ UFC/ml) dans le même volume.

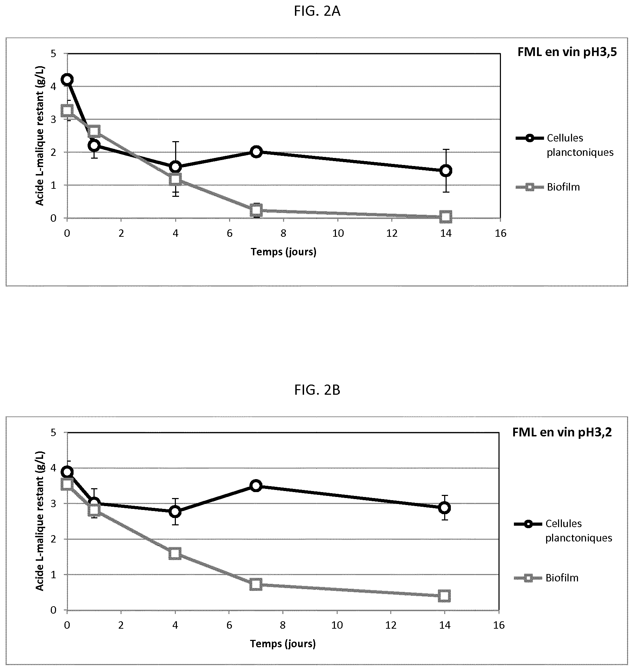

Les résultats sont présentés aux figures 2A et 2B.

Les conditions utilisées ici sont représentatives du milieu vin. A pH 3,5, la décarboxylation de l'acide malique par les cellules planctoniques est effective mais incomplète avec au bout de 14 jours une concentration résiduelle d'acide malique d'environ 1,5 g/l. Pour le vin synthétique à pH 3,2, les conditions apparaissent trop drastiques et la faible capacité fermentaire ne permet pas de consommer l'acide malique avec une concentration résiduelle en fin de fermentation de 3 g/l. Des dénombrements de ces bactéries aux différents temps de l'incubation ont permis de montrer une mortalité complète des cellules au bout des 4 jours ce qui explique l'arrêt de la consommation du malate à partir de ce temps pour les cellules planctoniques.

En revanche, les cellules de *O. oeni* cultivées sur coupon de bois sont capables d'achever la fermentation malolactique (consommation complète du malate) en 7 jours pour le vin synthétique à pH 3,5 et en 14 jours pour le vin synthétique à pH 3,2. La cinétique de dégradation est rapide et efficace et témoigne de la très bonne activité métabolique des bactéries en biofilm sur coupon de bois.

Cette expérience permet de conclure que la bactérie *O. oeni* cultivée en biofilm sur coupon de bois est métaboliquement active et capable de réaliser la fermentation malolactique dans des conditions mimant le vin et en présence de stress. Il faut retenir ici que :
- la culture en biofilm ne nécessite pas de pré-acclimatation des bactéries aux conditions de stress.
- les performances malolactiques de ces bactéries en biofilm sont supérieures à celles de bactéries communément cultivées en planctonique.

C'est le phénotype acquis par les bactéries au cours de la croissance en biofilm qui confère cette résistance qui aboutit à de meilleures performances pour utiliser le malate et donc réaliser la fermentation malolactique du vin dans un temps raccourci.

### 3. Influence de biofilm dans les échanges bois-vin :

### • Conditions de stress physico chimique : pH<3,5 ; éthanol ≥12% volumique

### • Résultats qualités organoleptiques:

Pour évaluer l'influence des biofilms dans les échanges bois-vin, 3 conditions ont été comparées: (i) des coupons de chêne immergés dans le vin appelée « chêne », (ii) une fermentation malolactique du vin réalisée par des bactéries planctoniques en présence de coupons de chêne appelée « FML+chêne », (iii) un biofilm de *O. oeni* sur les coupons de chêne capable de réaliser la FML du vin appelée « biofilm ».

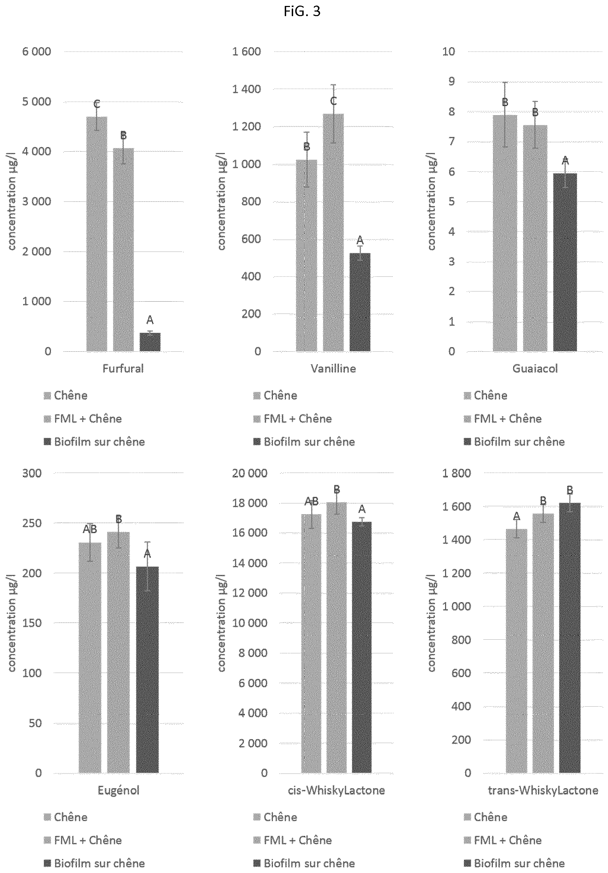

Au bout d'un mois d'élevage (contact entre le bois et le vin) six molécules aromatiques caractéristiques du bois ont été dosées dans les vins produits dans les 3 conditions ci-dessus ; ces molécules aromatiques sont le furfural (amande), la vanilline (vanille), le guaiacol (fumée), l'eugénol (clou de girofle) et les cis et trans whiskylactones (noix de coco). (Fig. 3)

Trois répétitions biologiques et deux techniques ont été effectuées.

Un test statistique ANOVA suivi du test post hoc Tukey HSD a été réalisé sur les données pour déterminer les différences significatives entre les groupes (les différences sont représentées par des lettres différentes : P=0,05)

La condition biofilm est différente des deux autres conditions pour le furfural, la vanilline et le guaiacol. Le biofilm a réduit l'extraction de ces trois arômes dans le vin.

De même, la condition « biofilm » donne une plus faible concentration d'eugénol et de cis whiskylactone que la condition « FML+chêne ».

La condition « biofilm » améliore l'extraction de la trans whiskylactone par rapport à la condition sans activité microbiologique « Chêne », mais n'est pas significativement différente de la condition « FML+chêne ».

L'analyse en composantes principales du jeu de données des dosages d'arômes montre que 84% de la variabilité est prise en compte par les deux composantes (Fig. 4). Il y a une forte corrélation de la première composante (61,31%) avec la vanilline, le furfural, le guaiacol et l'eugénol. Les trans et cis-whiskylactones sont fortement corrélées avec la composante 2 (22,72%). La séparation des données observée montre que les données de la condition « Biofilm » sont isolées des données des conditions « Chêne » et « FML + chêne » par la composante 1 qui représente 61,31% de la variabilité. Il y a donc une forte différence statistique entre ces conditions.

Le biofilm a donc modulé les transferts de molécules aromatiques du bois vers le vin.

La couleur du vin de ces mêmes échantillons a été analysée par spectrophotométrie (paramètres L* a* b*) (Fig. 5). Le vin blanc initial se colore par contact avec le bois, ce qui est montré par l'augmentation des paramètres a* (rouge) et b* (jaune) et la diminution de L* (clarté) entre « vin » et « chêne ». L'activité fermentaire des bactéries planctoniques dans « FML + chêne » ne modifie pas significativement ces paramètres.

En revanche, le « biofilm sur chêne » limite la coloration, le vin obtenu est moins orange, (a* et b*) et perd moins de clarté (L*).

Le biofilm a donc modulé les transferts de pigments du bois vers le vin.

### REFERENCES BIBLIOGRAPHIQUES

Cadahía, E., Fernández de Simón, B., and Jalocha, J. (2003). Volatile Compounds in Spanish, French, and American Oak Woods after Natural Seasoning and Toasting. J. Agric. Food Chem. 51, 5923-5932.
Díaz-Plaza, EM., Reyero, JR., Pardo, F., Alonso,GL., Salinas, MR. (2002). Influence of Oak Wood on the Aromatic Composition and Quality of Wines with Différent Tannin Contents. Journal of Agricultural and Food Chemistry 50 (9), 2622-2626
Fernandez de Simón B., Hernández, T., Cadahía, E., Dueñas, M., and Estrella, I. (2003). Phenolic compounds in a Spanish red wine aged in barrels made of Spanish, French and American oak wood. Eur Food Res Technol 216, 150-156.
Fernández de Simón, B., Sanz, M., Cadahía, E., Poveda, P., and Broto, M. (2006). Chemical Characterization of Oak Heartwood from Spanish Forests of Quercus pyrenaica (Wild.). Ellagitannins, Low Molecular Weight Phenolic, and Volatile Compounds. J. Agric. Food Chem. 54, 8314-8321.
Fernandez de Simon, B., Cadahia, E., Muino, I., Del Alamo, M., & Nevares, I. 2010. Volatile Composition of Toasted Oak Chips and Staves and of Red Wine Aged with Them. American Journal of Enology and Viticulture, 61(2), 157-165.
Nel, H.A., Bauer, R., Wolfaardt, G.M., and Dicks, L.M.T. (2002). Effect of bacteriocins pediocin PD-1, plantaricin 423, and nisin on biofilms of Oenococcus oeni on a stainless steel surface. American Journal of Enology and Viticulture 53, 191-196.
Pérez-Prieto, L.J., López-Roca, J.M., Martinez-Cutillas, A., Pardo Mínguez, F., and Gómez-Plaza, E. (2002). Maturing Wines in Oak Barrels. Effects of Origin, Volume, and Age of the Barrel on the Wine Volatile Composition. J. Agric. Food Chem. 50, 3272-3276.
Rieu, A., Aoudia, N., Jego, G., Chluba, J., Yousfi, N., Briandet, R., Deschamps, J., Gasquet, B., Monedero, V., Garrido, C., Guzzo Jean. (2014). The biofilm mode of life boosts the anti-inflammatory properties of Lactobacillus. Cell. Microbiol.

## Revendications

1. Procédé de préparation d'un vin comprenant le déclenchement de la fermentation par inoculation du vin avec des bactéries fermentaires du genre Oenococcus sous forme de biofilm.

2. Procédé de préparation selon la revendication 1 dans lequel le vin est en outre inoculée avec des levures fermentaires, les bactéries et levures fermentaires étant en combinaison sous forme d'un biofilm.

3. Procédé selon la revendication 1 dans lequel la fermentation est une fermentation malolactique et les bactéries fermentaires sont des bactéries lactiques sous forme de biofilm.

4. Procédé selon l'une des revendications 2 à 3 dans lequel les levures fermentaires sont choisies parmi les souches appartenant au genre *Sacharomyces, Schizosaccharomyces*, *Brettanomyces, Torulaspora, Candida, Metschnikowia, Kluyveromyces* ou leurs combinaisons.

5. Procédé selon l'une des revendications précédentes dans lequel les bactéries fermentaires sous forme de biofilm ou la combinaison des bactéries et levures fermentaires sous forme de biofilm sont inoculées sous une forme décrochée du support, ou sous une forme adhérente au support.

6. Procédé selon la revendication 5 dans lequel le support est choisi parmi le bois, de préférence les essences de bois utilisées comme contenant de produit liquide fermenté, le liège, l'acier inoxydable, le polystyrène, le silicone ou les composites de polyéthylène.

7. Procédé selon la revendication 5 ou 6 dans lequel les bactéries fermentaires sous forme de biofilm, ou la combinaison des bactéries et levures fermentaires sous forme de biofilm, avant inoculation, se présentent sous forme congelée, sous vide ou sous forme lyophilisée.

8. Procédé selon l'une des revendications précédentes dans lequel la quantité de bactéries inoculées varie de 10⁶ à 10⁷ UFC/mL.

## Patentansprüche

1. Verfahren zur Herstellung eines Weins, umfassend das Auslösen der Gärung durch Inokulieren des Weins mit Gärbakterien der Art Oenococcus in Form von Biofilm.

2. Verfahren nach Anspruch 1, wobei der Wein ferner mit Gärhefen inokuliert wird, wobei die Gärbakterien und -hefen in Kombination in Form eines Biofilms sind.

3. Verfahren nach Anspruch 1, wobei die Gärung eine malolaktische Gärung ist und die Gärbakterien Milchsäurebakterien in Form von Biofilm sind.

4. Verfahren nach einem der Ansprüche 2 bis 3, wobei die Gärhefen aus den Stämmen ausgewählt sind, die zu der Art *Sacharomyces, Schizosaccharomyces, Brettanomyces, Torulaspora, Candida, Metschnikowia, Kluyveromyces* oder deren Kombinationen gehören.

5. Verfahren nach einem der vorangehenden Ansprüche, wobei die Gärbakterien in Form von Biofilm oder die Kombination der Gärbakterien und -hefen in Form von Biofilm in einer vom Träger gelösten Form oder in einer am Träger anhaftenden Form inokuliert werden.

6. Verfahren nach Anspruch 5, wobei der Träger aus dem Holz, vorzugsweise den Holzarten, die als Behältnis für flüssiges Gärprodukt verwendet werden, dem Kork, dem rostfreien Stahl, dem Polystyrol, dem Silikon oder den Polyethylenkompositen ausgewählt wird.

7. Verfahren nach Anspruch 5 oder 6 wobei die Gärbakterien in Form von Biofilm oder die Kombination der Gärbakterien und -hefen in Form von Biofilm vor dem Inokulieren in tiefgefrorener Form, im Vakuum oder in lyophilisierter Form vorliegen.

8. Verfahren nach einem der vorangehenden Ansprüche, wobei die Menge inokulierter Bakterien von 10⁶ bis 10⁷ UFC/mL schwankt.

## Claims

1. Method for preparing a wine comprising the initiation of fermentation by inoculating the wine with fermenting bacteria of the genus *Oenococcus* in the form of a biofilm.

2. Preparation method according to claim 1, wherein the wine is further inoculated with fermenting yeasts, the fermenting bacteria and yeasts being combined in the form of a biofilm.

3. Method according to claim 1, wherein the fermentation process is a malolactic fermentation process and the fermenting bacteria are lactic bacteria in the form of a biofilm.

4. Method according to either claim 2 or claim 3, wherein the fermenting yeasts are chosen from the strains belonging to the genus *Saccharomyces, Schizosaccharomyces, Brettanomyces, Torulaspora, Candida, Metschnikowia, Kluyveromyces* or combinations thereof.

5. Method according to one of the preceding claims, wherein the fermenting bacteria in the form of a biofilm or the combination of fermenting bacteria and yeasts in the form of a biofilm are inoculated in a form that is separated from the support, or in a form that is adherent to the support.

6. Method according to claim 5, wherein the support is chosen from the group consisting of wood, preferably the wood varieties used as a fermented liquid product container, cork, stainless steel, polystyrene, silicone or polyethylene composites.

7. Method according to either claim 5 or claim 6, wherein the fermenting bacteria in the form of a biofilm, or the combination of fermenting bacteria and yeasts in the form of a biofilm, before inoculation, are present in a frozen form, under vacuum or in lyophilised form.

8. Method according to one of the preceding claims, wherein the quantity of inoculated bacteria varies from 10⁶ to 10⁷ CFU/mL.
